Europäisches Patentamt

European Patent Office

Office européen des brevets

(19)

(11) Publication number: 0 264 494
A1

# (12) EUROPEAN PATENT APPLICATION

(21) Application number: 86201847.0

(22) Date of filing: 23.10.86

(51) Int. Cl.4: G01N 33/00

(43) Date of publication of application:
27.04.88 Bulletin 88/17

(84) Designated Contracting States:
DE FR GB IT SE

(71) Applicant: Dumbeck, Robert F., Sr.
P.O. Box 548
Elgin Texas 78621(US)

(72) Inventor: Dumbeck, Robert F., Sr.
P.O. Box 548
Elgin Texas 78621(US)

(74) Representative: Petri, Stellan
c/o SAB NIFE AB Box 515
S-261 24 Landskrona(SE)

(54) Detecting and control of contaminated air in dwellings.

(57) Electronic instrumentation is provided for detecting the contamination of air in a dwelling to determine safeness for human occupancy. Thus, alarms may be sounded if the oxygen level falls below a safe value, or if there are other contaminants unsafe for human occupancy such as the presence of radon gas. Air passed through two flow paths is analyzed respectively by separate detectors and the signals compared. Air flowing in one path is safe for human consumption. In a portable instrument for use in-situ filtered air is used in one channel.

FIG 3

EP 0 264 494 A1

# DETECTION AND CONTROL OF CONTAMINATED AIR IN DWELLINGS

## Technical Field

This invention relates to detection and control of contaminated air in the interior living space of dwellings, and more particularly it relates to instrumentation for detecting variations from safe air standards and for control of the atmosphere inside dwellings.

## Background Art

There is now awareness that the air inside dwellings may become contaminated and unsafe for human occupation because of contaminants including tobacco smoke, radon gas and the like, and because of the lack of a safe level of oxygen. The Environmental Protection Agency in the USA has found extensive presence of radon gas seeping into dwellings at levels much higher than the standard of four picocuries per liter of air, and asserts that the lung cancer risks at higher levels are greater than those for extensive tobacco smoke or large numbers of chest X-rays.

Particularly in view of the backlash of increased energy costs in recent years, dwellings have had air leakages sealed about windows, doors, fireplaces, keyholes and the like. Thus it has not been uncommon to encounter asphyxia after expenditure of available oxygen within such dwellings to unsafe levels.

Because of decaying radioactive materials in the earth, radon gas tends to percolate through the earth about dwellings and seep into dwellings through walls and floors in contact with the earth. In order to detect such radon gas seepage, expensive instrumentation was required, and if found the conventional methods of protecting the homes have been expensive and temporary. Thus sealing of cracks or leakage through joints or about pipes requires expensive repairs and does not assure that all present and future entry positions are sealed. Also conventional venting methods for removing accumulated radon gas, usually in basements, are neither positive in action nor efficient in energy usage.

It is therefore an objective of this invention to provide simple instrumentation to detect unsafe air levels in dwellings, and to provide corrective apparatus for assuring air safety with efficient energy cost.

## Disclosure of the Inveniton

A simple and inexpensive electronic air contamination detector is provided sensitive to unsafe oxygen and radon gas levels as well as other kinds of air pollution which result in unsavory odors. The detector can quantitatively provide readings such as the percentage of oxygen in air with particularly sensitive response in the critical ranges of oxygen in air between the unsafe level of 17% and the 20.9% found in clean ambient air. Also the detector can quantitatively provide readings of radon gas presence at levels from four picocuries per liter of air upward.

Instrumentation may be portable for sensing the conditions in a dwelling, or installed in-situ with control apparatus for automatically controlling contaminated air and assuring safe air for human occupancy. The detection instrument compares the output signals of two electronic contamination detectors respectively located in a stream of air safe for human consumption and the air in a dwelling that need be monitored.

## Brief Description of the Drawings

The nature of the invention will be better understood from the following description as referenced to the accompanying drawings, in which:

Figure 1 is a circuit diagram, partially in block form, of a contamination detector afforded by this invention,

Figure 2 is a profile view of a preferred ionization detector element of the invention for analyzing air flow relative to the element,

Figure 3 is a block diagram illustrating the method of monitoring the quality of air in a dwelling by comparing the air to be monitored with air safe for human consumption,

Figure 4 is a diagrammatic sketch of a portable instrument useful in-situ in a dwelling to monitor air quality level, and

Figure 5 is a further sketch of a portable air monitor instrument.

## The Preferred Embodiment

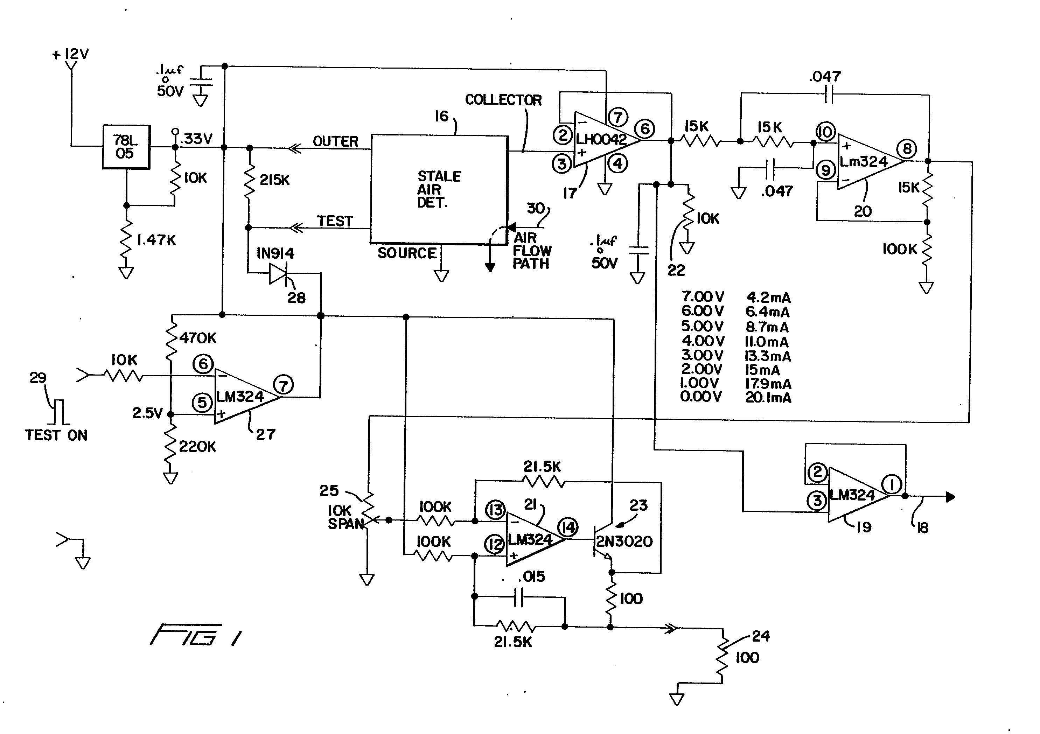

In the diagram of Figure 1, the typical circuit element values, voltages, and standard chip designation numbers are shown on the drawing in conventional format, except for the stale air detector 16. That element comprises a radiation driven ionization detector with a standardized micro Curie

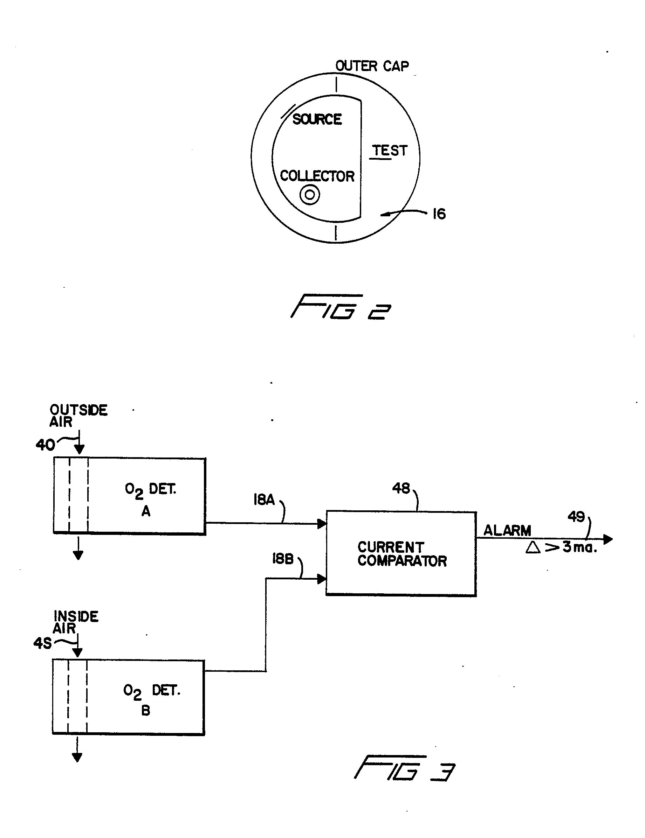

continuous radiation level derived from the self-contained isotope source AM241. Units of this type, as physically diagrammed in Figure 2, having an inner cavity which serves as the air flow path therethrough and the various connections itemized in Figure 1, are commercially available as model DSC.A3 from Amersham Corporation of Arlington Heights, Illinois, USA.

The present circuit is operated at an outer cap voltage of 9.33 volts as established by power supply chip 78L05. Output current from the collector is isolated at 17 by the low noise FET emitter follower operational amplifier LH0042 from the emitter circuit of which is branched two output signal paths. A first taken from the LM324 amplifier section 19 at output lead 18 is for control purposes or for comparison purposes as later described herein. The range of typical output voltages and currents at resistor 22 for corresponding signal variations in the stale air detector is set forth in the drawing table.

The remaining signal processing path through operational amplifiers 20, 21 feed the 2N3020 transmitter 23 to develop the output across the constant current configuration and 100 ohm load resistor 24. A span adjust potentiometer 25 is provided and the zero adjust is estblished by the voltage to the outer shell of the ionization detector, and may be selected by voltage supply 78L05.

A test circuit is provided by operational amplifier 27, which by way of diode 28 will in the presence of a test signal 29 serve to produce a standardized output, such as 13 milliamperes at output resistor 24, when the circuit normally operates. This will compare with a typical clean air reading output signal of 6 milliamperes obtained by means of passing clean ambient air of normal oxygen content through the stable air detector 16 by way of path 30.

This detector system serves to produce quantitative readings as a function of the amount of oxygen percentage in the air flow path through the ionization chamber detector element 16. Furthermore, it is made sensitive to and disproportionate in response to the critical percentages of oxygen in clean air (20.9%) through the range to the percentage of oxygen unacceptable in human dwellings (19.6% or under). In general, air will not support an open candle flame with less than about 16.7% of oxygen content, at about ambient temperature (25°C) and humidity (55%).

By comparison with commercially available oxygen analyzing equipment while varying the oxygen content of the measured air, it has thus been found that this circuit embodiment produced a set of comparative measurement points as follows when measuring air in a closed container having oxygen burned by candle flame:

| % $O_2$ | m/a |
|---------|-----|
| 20.9 | 5.9 |
| 20.1 | 9.0 |
| 19.7 | 12.7 |
| 18.3 | 13.6 |
| 17.0 | 14.0 |

In essence this forms a substantially double asymptotic exponentially shaped curve with the region between the asymptotes varying rapidly to produce the desired high sensitivity range between the normal oxygen level in ambient fresh air (20.9%) and the reduction of oxygen percentage to an unhealthy level (below 19.6%).

When the air in the box is replaced gradually by exhaled air from the human lungs, a substantially straight line characteristic is observed as the oxygen in the box decreases over the range of interest, to wit:

| % $O_2$ | m/a |
|---------|-----|
| 20.8 | 5.64 |
| 20.3 | 5.72 |
| 19.9 | 5.80 |
| 19.5 | 5.83 |
| 19.0 | 5.86 |
| 18.4 | 5.89 |

When odors such as from onions are introduced with exhaled air a similar result to that of the burning candle is achieved with increased sensitivity in the critical oxygen range. Odors alone without significant oxygen change react similarly to the response to exhaled human breath.

It is clear therefore that the instrumentation provides an ionization detector and circuit arrangement therefore producing a variable signal sensitive to oxygen detection in a critical range of about 21% to 17% of air contact, and furthermore that a combination of smoke, foul odors, etc. with reduced oxygen from human breathing consumption makes the instrument readings particularly sensitive in the critical region between the normal oxygen to air ratio of 20.9% to the range of about 17% which is below an acceptable level for human occupancy. Thus, the instrument is ideal for automatic control of venting of fresh air when the oxygen level in a house falls below an acceptable value, for example.

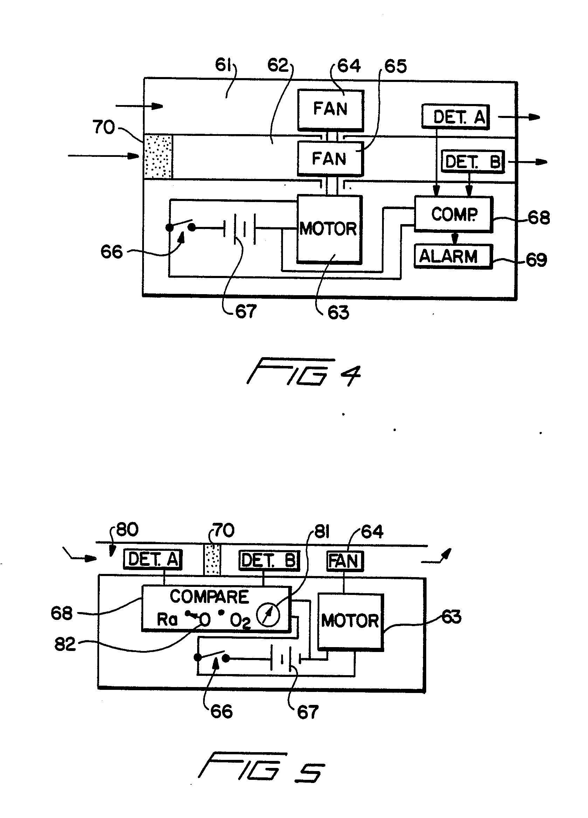

For constant surveillance of the air quality in a dwelling, two such stale air detector units may be coupled as shown in Figure 3. Thus, oxygen detector unit A may receive a flow of outside clean ambient air, 40, while oxygen detector unit B receives a flow of inside air, 45, from the dwelling. A current comparator 48 then can sound an alarm at 49 (or produce a control signal) whenever the current magnitude from the inside air produces a difference greater than about three milliamperes from the clean ambient outside air, presumably with 20.9% oxygen, or reaching a level slightly above the unacceptable 19.6% oxygen level as set forth in the foregoing operational characteristic table.

Thus, the foregoing invention provides inexpensive reliable means of detecting stale air for regulating and controlling the air in a dwelling in a new and novel manner for an acceptably healthy environment.

It is noted also that the broad band capabilities of the stale air detector also substantially follows the sensitivity of the human nose to odors and will provide non-ambiguous readings fully acceptable to control of the air in a dwelling for other ionization conditions imposed by smoke, cooking and food odors and the like, which need be eliminated from the tightly sealed dwelling commonplace at present. The control of flow of fresh air into the dwelling upon detection of an alarm condition may proceed either manually or automatically from a system controlled in response to the electronic alarm output signals made available.

This detector also is sensitive to the presence of radon gas at low levels such as the four picocuries pere liter of air which is set by the Environmental Protection Agency of USA as the maximum tolerable limit for presence in dwellings to avoid significant dangers of lung cancer. The presence of radon gas is thus a factor which will result in an alarm corresponding to that of a deficiency of oxygen.

It is desirable to provide a portable low cost instrument that can detect the quality of air in a dwelling and which in fact can detect the presence of radon gas, which has a tendency to accumulate in basements or areas of a dwelling close to walls or floors in contact with the earth. For this purpose, reference is made to the Figure 4 embodiment.

The housing 60 is shown to encompass a portable air quality detector operable in the foregoing manner. It is readily within the skill of those in the art to calibrate the instrument for the presence of either deficiency of oxygen or the presence of radon at levels dangerous to human health.

Since a portable instrument for use inside a dwelling does not have access to a convenient source of outside fresh air, the air being tested flows through one channel 61, and the comparative air is filtered to provide contamination free air flowing through a second channel 62. To maintain balance, the air flow is designed to be of equal volume in both channels, so that detectors A and B will be at the same temperature in similar air flow paths and provide a differential reading solely from the difference in air quality in the two paths.

A single motor 63 in this respect has two rotary fan portions 64, 65 which draw air respectively through the instrument channels 61, 62 in the direction of the arrows, when switch 66 is on, as energized from battery 67. The comparator circuit 68, as hereinbefore described, operates with detectors A and B to sound an alarm 69, or give a meter reading, etc.

In order to compare in-situ air with an equivalent comparative reference to the fresh atmospheric air standard hereinbefore discussed, the air in channel 62 is filtered through filter 70, which eliminates pollutants including tobacco, pollen, dust, bathroom odors, smoke, radon gas and the like. One preferable filter is obtainable from Westclox, Talley Industries division of General Time, Technology Park, Atlanta, Norcross, Georgia, USA. An acceptable filter is described in US Patent 4, 534,775. Activated charcoal will remove most pollutants including radon gas.

It may be seen therefore that the instrument calibration or meter reading will signify by the differences in air quality between channels 61 and 62 as processed through detectors A and B and comparator 68 the quality of air inside the dwelling. Concentrations of poor quality air in basements or near underground walls, particularly if around damp areas, sump pumps or cracks in the foundation would tend to indicate the presence of radon gas. Other unsafe areas or conditions of air quality such as deficiency of oxygen are also detectable with the same instrumentation in the manner hereinbefore described.

In Figure 5 is a further simplified version of the portable air contamination instrument wherein one flow pipe 80 has both detectors A and B therein separated by the filter 70. Thus, the safe air reference at detector B is compared with the ambient sampled air at detector A to determine the contamination level.

The configuration of Figure 5 may be calibrated separately for detection of radon and oxygen by choice of resistors to read out correspondingly on meter 81 a contamination index. Switch 82 chooses the desired detection mode. The filter 70 removes radon and such contaminants as smoke and odors. Enough $CO_2$ may be removed for proper sampling inside dwellings of a safe oxygen ratio. Control of the contaminant removal may result both as a function of the air flow rate provided by fan 64 and by the nature of the filter 70.

Thus, the portable instrument of Figures 4 and 5 provides a simple inexpensive means of sampling air contamination in a dwelling to determine the degree of safety for human occupation.

Having therefore set forth improvements in the art and available novel means and methods not heretofore in the art, those features of novelty believed descriptive of the nature and spirit of my invention are set forth with particularity in the following claims.

## Claims

1. A contaminated air detection system for monitoring the quality of air in a dwelling, **characterized** by:
two electronic ionization detectors, each sensitive to the flow of air thereby for indicating by an electronic signal the presence of contamination in the air flowing by the respective detector,
means for flowing air of a quality safe for human consumption by a first said detector to generate a first signal therefrom,
means for flowing air to be monitored by the other of said detectors for generating a second signal therefrom, and
signal comparison means coupled to compare the signals from the two detectors to produce an indication of the safeness of the quality of the air being monitored for human consumption.

2. A system as defined in claim 1, **characterized** by:
a portable instrument for sampling air in-situ in a dwelling to flow air at the sampling site by both said detectors through separate flow paths, and
filter means for removing air contamination from one path so that the quality of the air in the dwelling is established by the comparison of the signals from the two detectors.

3. A system as defined in claim 2, **characterized** by an air blower in the instrument for forcing air through the separate flow paths.

4. A system as defined in claim 1, **characterized** in that said signal comparison means has output signal generation means providing a signal derived as a function of the percentage of oxygen in the air being monitored.

5. A system as defined in claim 4, **characterized** in that said signal comparison means has circuit means for producing a significant difference in signal characteristics for changes of oxygen content in the range of 17% to 21%.

+ 12V

.1µf 50V

78L 05

.33V

10K

1.47K

OUTER

215K

TEST

1N914

28

STALE AIR DET.

16

COLLECTOR

SOURCE

30

AIR FLOW PATH

2 ⑦ ⑥
③ LH0042 ④
17

15K 15K ⑩
+
⑨ Lm324 ⑧
.047
20

15K

100K

.047

.1µf 50V

10K

22

| 7.00 V | 4.2 mA |
|--------|--------|
| 6.00 V | 6.4 mA |
| 5.00 V | 8.7 mA |
| 4.00 V | 11.0 mA |
| 3.00 V | 13.3 mA |
| 2.00 V | 15 mA |
| 1.00 V | 17.9 mA |
| 0.00 V | 20.1 mA |

470K

10K

29

TEST ON

⑥
⑤ LM324 ⑦
2.5V +
27

220K

25 10K SPAN

100K ⑬ 21
⑫ LM324 ⑭
100K +

21.5K

23 2N3020

.015

21.5K

100

24 100

② LM324 ①
③ 19
18

FIG 1

0 264 494

OUTER CAP

SOURCE

TEST

COLLECTOR

16

*FIG 2*

OUTSIDE
AIR

40

$O_2$ DET.
A

18A

48

CURRENT
COMPARATOR

ALARM

49

$\triangle > 3$ ma.

18B

INSIDE
AIR

4S

$O_2$ DET.
B

*FIG 3*

61  62  64  65

FAN

DET. A

70

FAN

DET. B

66

MOTOR

COMP.  68

ALARM  69

67  63

FIG 4

80  70  81  64

DET. A  DET. B  FAN

68

COMPARE
Ra O O₂

MOTOR  63

82

66  67

FIG 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | STAUB-REINHALTUNG DER LUFT, vol. 43, no. 1, January 1983, pages 25-28, Speyer/Rhein; M. LEHTIMÄKI et al.: "Über das Verhalten des Radons in geschlossenen Raümen" * abstract; figure 2 * | 1 | G 01 N 33/00 |
| Y | GB-A-1 579 144 (BRUNSWICK CORP.) * page 1, lines 26-35; page 3, lines 4-6; page 5, lines 45-53 * | 1 | |
| A | US-A-4 116 042 (A. JENKINS et al.) * column 1, lines 56-66 * | 1 | |
| A | CH-A- 181 580 (GEBR. SULZER AG) * whole document * | 1 | TECHNICAL FIELDS SEARCHED (Int. Cl.4) |
| A | WO-A-8 604 708 (NORSK VIFTEFABRIKK) * page 7, lines 11-17, claims 1, 2 * | 1 | G 01 N 33/00 G 01 N 27/00 G 08 B 17/00 F 24 F 11/00 |
| A | GB-A-2 054 153 (COAL INDUSTRY PATENTS, LTD.) * page 1, lines 35-51, 117-124 * | 1,4,5 | |

The present search report has been drawn up for all claims

| Place of search | Date of completion of the search | Examiner |
|---|---|---|
| BERLIN | 23-06-1987 | BRISON O.P. |